# EUROPEAN PATENT APPLICATION

(11) **EP 4 420 725 A2**
(43) Date of publication of application: **28.08.2024**
(21) Application number: 24173363.3
(22) Date of filing: 27.03.2013
(51) Int. Cl.: A61P 27/02

(54) **OCULAR TREATMENT SOLUTIONS, DELIVERY DEVICES AND DELIVERY AUGMENTATION METHODS**

(30) Priority: 29.03.2012 US 201261617339 P
(62) Divisional of application: 13767439.6
(71) Applicant: Epion Therapeutics, Inc., Burlington, MA 01803 (US)
(72) Inventor: RUBINFELD, Roy, S, Bethesda, 20817 (US); HARTMAN, Raymond, A, deceased (US); TRATTLER, Bill, Miami, 33176 (US)
(74) Representative: Dehns

(57) **Abstract**

A riboflavin solution for use in corneal strengthening treatment and the like has a higher concentration of riboflavin and may include other components for increasing corneal cross-linking. The solution can be oxygen saturated for improved oxygen supply to the cornea. A preparation device and method for buffing the corneal epithelium allows better penetration of the epithelium by riboflavin solution when subsequently applied to the eye.

## Description

### CROSS-REFERENCE

This application claims priority to U.S. Provisional Application No. 61/617,339, filed March 29, 2012, which is incorporated herein by reference in its entirety.

### BACKGROUND

Collagen cross-linking is a parasurgical treatment for multiple ophthalmic disorders. In some cases, collagen cross-linking may also be combined with other treatments to improve corneal strength or optical refraction. Treatment methods include mini asymmetric radial keratotomy, corneal ring segment inserts, or topography-guided laser. Corrective lenses are normally required after these treatments, but with smaller, more normalized prescriptions. Increased corneal symmetry allows for more comfortable contact lens wear, often of daily disposable lenses. Collagen crosslinking limits deterioration of vision, increases unaided and uncorrected vision, and may reduce the need for corneal transplantation.

### SUMMARY

Disclosed herein, in certain embodiments, is a composition for promoting photochemical cross-linking in eye tissue, the composition comprising: 0.2% to 10.0% by weight riboflavin in an aqueous carrier. In some embodiments, the composition comprises 0.2% to 5.0% by weight riboflavin. In some embodiments, the composition comprises 1.0% to 2.0% by weight riboflavin. In some embodiments, the composition further comprises 0.001% to 6.0% sodium iodide or catalase by weight. In some embodiments, the iodide is in the ionized (I⁻) form. In some embodiments, the composition has a pH from about 7.0 to about 8.4. In some embodiments, composition is a dry powder. In some embodiments, the composition is stored in an oxygen permeable plastic container. In some embodiments, the oxygen permeable plastic container comprises polyurethane or low density polyethylene. In some embodiments, the composition further comprises pressurized oxygen. In some embodiments, the composition comprises 350 to 500 Torr oxygen. In some embodiments, the composition further comprises a reducing agent. In some embodiments, reducing agent comprises one or more of sodium thiosulfate, vitamin C, and sodium bisulfate.

Disclosed herein, in certain embodiments, is a method for enhancing photochemical cross-linking in eye tissue, comprising: introducing to the eye tissue a composition comprising 0.2% to 10.0% by weight riboflavin in an aqueous carrier. In some embodiments, the composition comprises 0.2% to 5.0% by weight riboflavin. In some embodiments, the composition comprises 1.0% to 2.0% by weight riboflavin. In some embodiments, the method further comprises administering 0.001% to 6.0% by weight sodium iodide or catalase, artificial tears, or any combinations thereof. In some embodiments, the composition has a basic pH. In some embodiments, the basic pH is from about 7.0 to about 8.4. In some embodiments, the composition is a dry powder. In some embodiments, the method further comprises keeping the composition in an oxygen permeable plastic container. In some embodiments, the oxygen permeable plastic container comprises polyurethane or low density polyethylene. In some embodiments, the method further comprises placing the oxygen permeable plastic container in an oxygen chamber to be supplied and saturated with pressurized oxygen. In some embodiments, the method further comprises saturating the composition with oxygen. In some embodiments, the composition is saturated with oxygen to have an oxygen content of 350-500 Torr oxygen. In some embodiments, the composition further comprises a reducing agent. In some embodiments, the reducing agent comprises one or more of sodium thiosulfate, vitamin C, and sodium bisulfate.

Disclosed herein, in certain embodiments, is a method of applying an ophthalmic composition to an eye, comprising applying the ophthalmic composition after buffing the epithelium of an eye. In some embodiments, the method comprises buffing the epithelium of the eye by contacting the eye with a sponge device comprising a sponge. In some embodiments, the method comprises buffing the epithelium of the eye by rubbing the eye with a sponge device. In some embodiments, the method comprises buffing the epithelium of the eye by rubbing the eye with a sponge device in a circular pattern. In some embodiments, the method comprises removing lipids, mucus and microvilli. In some embodiments, the sponge is dry or comprises an ophthalmic composition. In some embodiments, the sponge device comprises a riboflavin composition, artificial tears, or a combination thereof. In some embodiments, the sponge is round. In some embodiments, the method further comprises applying the ophthalmic composition with a second sponge or sponge device. In some embodiments, the method further comprises placing the second sponge or sponge device over the eye to act as a depot or reservoir for the ophthalmic composition.

Disclosed herein, in certain embodiments, is a sponge device comprising a round sponge which operatively covers all or a portion of the eye. In some embodiments, the size of the sponge does not exceed the size of the eye. In some embodiments, the diameter of the sponge is about 3mm- about 12mm. In some embodiments, the sponge device further comprises 0.2% to 10.0% by weight riboflavin in an aqueous carrier, and optionally, sodium iodide, catalase, artificial tears, or any combinations thereof. In some embodiments, the sponge further comprises microfilaments. In some embodiments, the sponge is made of cellulose or polyvinyl acetate. In some embodiments, the sponge device comprises a handle operatively connected to the sponge. In some embodiments, the sponge device does not comprise a handle.

Disclosed herein, in certain embodiments, is a sponge device for use in introducing microabrasions into an eye, comprising a sponge having a surface shaped for rubbing across the surface of an eye. In some embodiments, the sponge has little or no risk of disrupting or perforating the epithelium. In some embodiments, the sponge is round. In some embodiments, the sponge does not have a pointed tip. In some embodiments, the sponge does not have sharp edges. In some embodiments, the sponge has a pointed tip. In some embodiments, the sponge is spear-tipped or arrow shaped. In some embodiments, the sponge has a feathered tip. In some embodiments, the sponge has a beveled edge. In some embodiments, the sponge has a 10 to 80 degree beveled edge. In some embodiments, the sponge has flat edges. In some embodiments, the sponge is made of a cellulose or polyvinyl acetate. In some embodiments, the sponge further comprises microfilaments. In some embodiments, the microfilaments are vertically oriented. In some embodiments, the microfilaments are horizontally oriented. In some embodiments, the microfilaments are not arranged in an ordered pattern. In some embodiments, the microfilaments are arranged in a disordered pattern. In some embodiments, the sponge does not comprise microfilaments. In some embodiments, the sponge device further comprises a handle operatively connected to the sponge. In some embodiments, the sponge device does not comprise a handle. In some embodiments, the sponge device further comprises 0.2% to 10.0% by weight riboflavin in an aqueous carrier, and optionally, sodium iodide, catalase, artificial tears, or any combinations thereof.

Disclosed herein, in certain embodiments, is a riboflavin solution which has a higher concentration of riboflavin than current riboflavin solutions used in photochemical treatment of the cornea, specifically 0.2 wt. % to 5.0 wt. % riboflavin in an aqueous carrier solution. In some embodiments, the solution contains about 0.5 wt. % riboflavin. In some embodiments, the solution contains about 1.0 wt. % riboflavin or about 2.0 wt. % riboflavin. In some embodiments, the higher concentration of riboflavin increases corneal cross-linking if associated with higher amounts of oxygen in the cornea. In some embodiments, the riboflavin solution also contains additional components for improved cross-linking, such as iodide ion or catalase enzyme. In some embodiments, the riboflavin solution includes 0.001% to 6.0% by weight sodium iodide, with the iodide kept in the ionized (I⁻) form by suitable control of the basic solution to a pH of 7.0 to 8.4 with no acids or other oxidizing agents. Iodide ion is a sodium peroxide reducing agent and acts to convert hydrogen peroxide into water and oxygen. Thus, inclusion of iodide ion in the riboflavin solution can reduce toxic hydrogen peroxide in the eye and also increase the amount of oxygen available for cross-linking.

Disclosed herein, in certain embodiments, is an oxygen saturated riboflavin or tear solution for topical application to the eye, which can be used for various eye conditions as well as in conjunction with photochemical cross-linking treatment.

Further disclosed herein, are methods of saturating a riboflavin or tear solution for topical application to the eye with oxygen. In some embodiments, vials of riboflavin solution or tear solution in oxygen permeable plastic containers, such as polyurethane or low density polyethylene containers, are placed in an oxygen chamber having a pressurized oxygen inlet and an exhaust outlet. In some embodiments, after placing the containers in the oxygen chamber, the inlet is connected to a supply of pressurized oxygen, and oxygen is run through the container and then turned off, such that the one way inlet and outlet valves in the oxygen inlet and exhaust outlet are closed and the chamber is sealed. In some embodiments, the vials are left in the oxygen chamber for an extended period of time to allow oxygen to permeate the walls of the plastic vials and saturate the contained solutions. In some embodiments, at the end of the extended time period or intermittently, pressurized oxygen is again run through the chamber to refresh the oxygen or flush out other gases. In some embodiments, the oxygenated solutions are removed from the chamber when needed, and are used within one hour of removal from the chamber to reduce oxygen loss.

Disclosed herein, in certain embodiments, are sponges that improve the penetration of riboflavin or other ophthalmic drugs through the epithelial barrier to avoid the surgical complications that arise from de-epithelialization, which in some embodiments result in more disruption of the tissue than is necessary for the procedure to be effective. In some embodiments, the sponge is sterile. The reduction in patient discomfort combined with more rapid restoration of visual acuity make a transepithelial procedure better for the patient. In some embodiments, the sponge is a dry sponge with a shaped edge. In some embodiments, sponges that serve this function also incorporate microfilaments to enhance epithelial permeability by gently removing lipids, mucus, and dead surface epithelial cells. In some embodiments, the sponges have little or no risk of disrupting or perforating the epithelium resulting in as little disruption of the epithelium as possible while still increasing penetration of the riboflavin or other solution into the deeper layers of the cornea without disrupting or causing epithelial defects in the corneal surface.

Additionally disclosed herein, in certain embodiments, is a sponge composed of a material designed to increase the permeability of the epithelium and simultaneously act as a reservoir for loading the cornea with an ophthalmic solution (e.g., riboflavin solution). In some embodiments, the sponge is sterile. In some embodiments, the sponge is round. In some embodiments, sponges that serve this function also incorporate microfilaments to enhance epithelial permeability by gently removing lipids, mucus, and dead surface epithelial cells. In some embodiments, the sponges have little or no risk of disrupting or perforating the epithelium resulting in as little disruption of the epithelium as possible while still increasing penetration of the riboflavin or other solution into the deeper layers of the cornea without disrupting or causing epithelial defects in the corneal surface. In some embodiments, this sponge is packaged along with a blunt plastic shaft or other tool to assist in "twirling" this sponge around on the cornea or sclera or other part of the eye to be treated in an effort to enhance penetration of the riboflavin or other solution into the eye.

Further disclosed herein, in certain embodiments, is a method of using a sponge disclosed herein to improve the penetration of riboflavin or other ophthalmic drugs through the epithelial barrier to avoid the surgical complications that arise from de-epithelialization, which in some embodiments result in more disruption of the tissue than is necessary for the procedure to be effective. The reduction in patient discomfort combined with more rapid restoration of visual acuity make a transepithelial procedure better for the patient. In some embodiments, the sponge is rubbed gently over the surface of the eye in a circular pattern after application of a topical anesthetic, in order to prepare the epithelium for improved penetration of riboflavin or other solutions. In some embodiments, this forms subtle or very fine microscratches on the epithelium to allow the riboflavin solution to penetrate through the epithelium into the cornea more easily. In some embodiments, the method removes lipids, mucus and microvilli. In some embodiments, the method has little or no risk of disrupting or perforating the epithelium resulting in as little disruption of the epithelium as possible while still increasing penetration of the riboflavin or other solution into the deeper layers of the cornea. In some embodiments, the method further comprises applying an ophthalmic solution (e.g., a riboflavin solution) to the eye in any suitable manner. In some embodiments, the ophthalmic solution is applied through a second, softer sponge placed over the eye to act as a depot or reservoir for the ophthalmic solution, since the tear film itself is only about 5 microns thick, which offers only a very small reservoir of riboflavin solution.

Other features and advantages of the present disclosure will become more readily apparent to those of ordinary skill in the art after reviewing the following detailed description and accompanying drawings.

### BRIEF DESCRIPTION OF THE DRAWINGS

The details of the present disclosure, both as to its structure and operation, may be gleaned in part by study of the accompanying drawings, in which like reference numerals refer to like parts, and in which:
FIG. 1 exemplifies a method of saturating topical ocular solutions in permeable plastic vials with oxygen according to some embodiments of the disclosure;
FIG. 2 exemplifies a front elevation view of a sponge for use in preparing the corneal epithelium for subsequent application of an ocular treatment solution to the eye according to some embodiments of the disclosure.
FIG. 3A and 3B illustrate exemplary edge shapes for the sponge.
FIG. 4 illustrates an exemplary use of the sponge of FIG. 2 to buff the corneal surface in a swirl action.
FIG. 5 is an exemplary illustration of a spear-tipped sponge which may be used with some embodiments of the disclosure.
FIG. 6 is an illustration of a sponge with a beveled tip edge which may be used with the embodiments of the disclosure.
FIG. 7 is a photograph of the use of a round sponge.
FIG. 8 is an exemplary illustration of a spear-tipped sponge with vertically-aligned microfilaments which may be used with some embodiments of the disclosure.
FIG. 9 is an exemplary illustration of a spear-tipped sponge with horizontally-aligned microfilaments which may be used with some embodiments of the disclosure.
FIG. 10 is an exemplary illustration of a sponge with a 10 to 80 degree beveled edge which may be used with some embodiments of the disclosure.

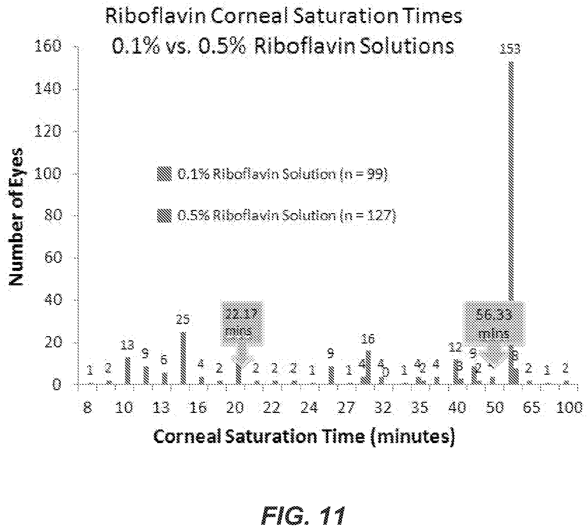

FIG. 11 illustrates corneal saturation time (in minutes) of a 0.1% riboflavin solution and a 0.5% riboflavin solution.

### DETAILED DESCRIPTION

Certain embodiments as disclosed herein provide for ocular treatment solutions, delivery devices for such solutions, and delivery augmentation methods. For example, according to embodiments of the disclosure, a device and method for preparing the corneal epithelium for better penetration of riboflavin solution into the cornea . Other embodiments include improved riboflavin solutions and methods of producing oxygen saturated tear solutions and riboflavin solutions.

A popular treatment of corneal diseases, including keratoconus, post-LASIK ectasia, and pellucid marginal degeneration, involves the removal of the epithelium followed by administration of a riboflavin solution and irradiation by ultraviolet-A light. Riboflavin acts as a photosensitizer and facilitates the cross-linking of stromal collagen fiber which prevents further disease progression. However, the removal of the epithelium carries numerous risks for the patient, including post-operative pain, infection risk, delayed wound healing, stromal haze, and herpetic keratitis. Therefore, it would be safer to treat the patient without having to surgically remove the epithelium. Unfortunately, there are many obstacles to this approach because the epithelium prevents the cornea from absorbing the riboflavin solution. Riboflavin is a large molecule that does not penetrate the epithelium well. This invention discloses novel compositions and methods for treating corneal diseases with riboflavin solutions without removing the epithelium.

An additional problem with the current treatment of corneal diseases with Riboflavin solutions is that hydrogen peroxide is produced in the eye as a side-product. Hydrogen peroxide is damaging to the eye. This invention discloses a composition and methods for minimizing this damaging side-product.

After reading this description it will become apparent to one skilled in the art how to implement the disclosure in various alternative embodiments and alternative applications. However, although various embodiments of the present disclosure will be described herein, it is understood that these embodiments are presented by way of example only, and not limiting. As such, this detailed description of various alternative embodiments should not be construed to limit the scope or breadth of the present disclosure as set forth in the appended claims.

### Riboflavin Solutions

According to embodiments of the disclosure, an ocular riboflavin solution for use in corneal treatment such as photochemical cross linking contains 0.2 wt. % to 5.0 wt. % riboflavin in an aqueous carrier solution, or up to 10.0% in some cases. In some embodiments, the solution contains about 0.5 wt. % riboflavin. In other examples, the solution contains about 1.0 wt. % riboflavin or about 2.0 wt. % riboflavin. The higher concentration of riboflavin can increase corneal cross-linking if associated with higher amounts of oxygen in the cornea. In many embodiments, the riboflavin solution is placed in an actinic glass or UV and visible light protected plastic containers, to avoid activation of the riboflavin by ambient light.

In some embodiments, the riboflavin is riboflavin-5'-phosphate. In some embodiments, the riboflavin solution is hypotonic with the eye. In some embodiments, the riboflavin solution comprises about 0.4% - about 0.9 % of a salt. In some embodiments, the riboflavin solution comprises about 0.4% - about 0.9% of a NaCl. In some embodiments, the riboflavin solution has a neutral pH, for example about 7.

In other examples, the riboflavin solution further comprises additional components for increased cross-linking, such as iodide ion or catalase enzyme. In some embodiments, the riboflavin solution includes 0.001% to 6.0% by weight sodium iodide, with the iodide kept in the ionized (I⁻) form by suitable control of the basic solution to a pH of 7.0 to 8.4 with no acids or other oxidizing agents. The solution contains sufficient iodide ion and/or catalase to decompose 20 micromolar hydrogen peroxide within one minute.

Iodide ion is a sodium peroxide reducing agent and acts to convert hydrogen peroxide into water and oxygen. Thus, inclusion of iodide ion in the riboflavin solution reduces toxic hydrogen peroxide in the eye and also increases the amount of oxygen available for cross-linking.

In some compositions, reducing agents such as sodium thiosulfate, vitamin C or sodium bisulfate are added to the solution to reduce any iodine formed back to iodide ions. These additives are FDA approved non-active ingredients for ophthalmic solutions.

When catalase and/or iodide ion is included in the riboflavin solution, the resulting mixture is in some embodiments lyophilized for greater stability during storage and transport, and then reconstituted with a dilute hydrogen peroxide mixture, for example in the range of 100 to 500 micromolar. The advantage of this method is that the added hydrogen peroxide is converted into molecular oxygen in the reconstituted riboflavin solution, and high partial pressures of oxygen can be achieved. For example using a solution of 400-micromolar hydrogen peroxide to reconstitute lyophilized riboflavin mixture containing catalase or iodide anion provides enough oxygen to saturate the solution to 300 mm of Hg of oxygen. This provides double the capacity of available oxygen in the solution compared to an atmospherically exposed riboflavin solution. The riboflavin and peroxide solutions should be placed in actinic glass or UV and visible light protected gas impervious plastic containers if not intended for immediate use.

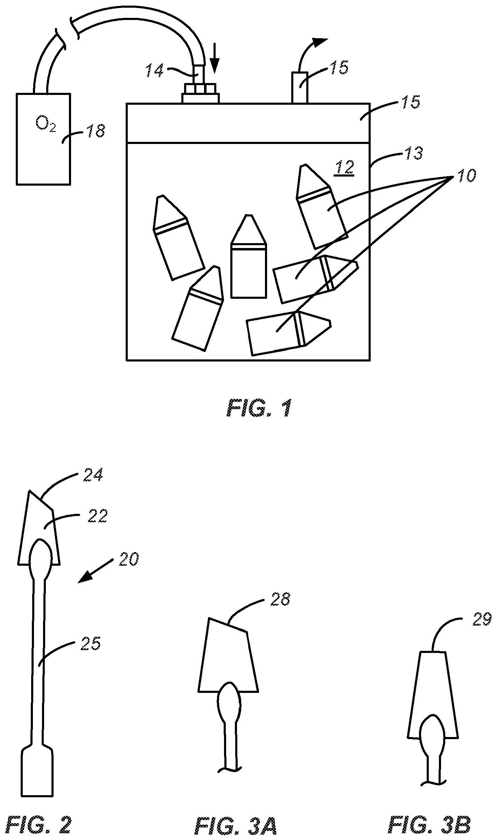

FIG. 1 illustrates an alternative apparatus and method for saturating an ocular treatment solution with oxygen immediately prior to application to the eye according to embodiments of the disclosure. Adding oxygen to the solution has many advantages both for topical treatments and solutions intended to soak into the underlying tissues of the cornea. In some embodiments, this method is used to prepare oxygen saturated riboflavin solutions as well as oxygen saturated tear solutions for use during or after eye treatment or surgery.

In this method, vials 10 of riboflavin solution or tear solution in gas permeable plastic containers are placed in a pressurized oxygen chamber 12 for an extended time period to allow oxygen to pass through the walls of the containers and saturate the contained solutions. Tear solutions are often packaged in containers of different plastics with different permeability to oxygen, such as low density polyethylene (LDPE). For example, Systane^{®} Balance is bottled in LDPE (low density polyethylene) which is a highly oxygen permeable plastic, and the bottle has a cap made of polystyrene. In some embodiments, any containers or vials which are oxygen permeable are used in this method.

The apparatus in some embodiments comprises a jar 13 having a pressurized oxygen inlet 14 and an exhaust outlet 16 in removable lid 15 as illustrated in FIG. 1, but other pressure chambers are used in alternative embodiments. In the example of FIG. 1, lid 15 comprises a screw top lid sealed with a rubber gasket or the like to seal chamber 12. A suitable one way check valve in the inlet for gas flow into the chamber and a second one way check valve at the outlet for gas flow out of the chamber. Once the lid is replaced and tightened to seal the chamber, the inlet 14 is connected to a supply 18 of pressurized oxygen, such as an oxygen cylinder. In some embodiments, the regulator on the oxygen cylinder set to about 2 psig (or 16.7 lbs psia).

The inlet check valve to the chamber then opens automatically, and oxygen flows into chamber 12, while excess air flows out through outlet 16, opening the outlet check valve. After allowing oxygen to flow through the chamber 12 for a sufficient time period to exhaust all air from the chamber and replace it with pressurized oxygen, typically 10 to 15 seconds, the oxygen supply is disconnected. The inlet and outlet check valves then close automatically, and the jar can be stored for a sufficient time to allow oxygen to permeate the walls of the plastic vials and saturate the contained solutions. After 12 or 24 hours, pressurized oxygen is again connected to the chamber inlet to re-purge the chamber. For best results, the vials are left in the oxygen filled chamber for a minimum of 24 to 48 hours. The oxygenated solutions may have 350 to 500 Torr oxygen content. This is two to three times higher than the oxygen content of normal tear films on the eye which have been equilibrated with atmospheric oxygen. The higher content of oxygen allows for faster and greater oxygen delivery to the stroma. The containers of oxygenated tear solutions or riboflavin solutions are removed from the chamber when needed, and are applied to the eye within one hour of removal from the chamber to reduce oxygen loss.

In a conventional ocular photochemical treatment procedure with standard riboflavin solutions, the stroma becomes hypoxic within a few seconds after irradiation has commenced, due to the relatively slow oxygen uptake rate of the cornea. The maximum corneal oxygen uptake (COU) rate for an open eye is about 10 microliters-O₂ cm²/hour or about 1.2 × 10⁻¹⁰ moles- O₂ cm²/second. Energy being applied at 3.0 mw/cm² consumes oxygen in the photochemical process at a rate 30 times greater than the maximum reported COU. Because oxygen is replenished to the cornea from the overlying tear film, and the tear film is only 5 microns thick on average (1/100th of the stromal thickness), it would require the oxygen content of 100 individual fully oxygenated tear films to replenish all the oxygen in a 500-micron thick stroma. Replenishment of the oxygen in a tear film either requires blinking or re-equilibration of the tear film from the atmosphere. Blinking is usually difficult during photochemical cross-linking because the eyelids are usually held open by a clamp during the procedure. The literature reports that an unoxygenated tear film can require up to 60 seconds to reestablish its level of dissolved oxygen from the atmosphere. This leads to a continuous hypoxic state of the stroma during irradiation, and creates the conditions for high Type I hydrogen peroxide production in the stroma. In some cases, other eye stress conditions due to eye diseases or injuries also result in production of hydrogen peroxide.

In many embodiments, the application of highly oxygenated solutions to the eye avoids or reduces the problem of reduced corneal oxygen during photochemical treatment by adding extra oxygen to the eye. In some embodiments, oxygenated solutions are also beneficial for healing purposes by reducing the amount of toxic hydrogen peroxide in the eye. In some embodiments, application of oxygenated riboflavin and tear solutions to the eye for photochemical treatment take place before treatment with a selected wavelength of light commences, during treatment, or both.

In alternative embodiments, the topical solutions comprise lipid or oil-based fluids that are pre-oxygenated at high oxygen partial pressures (150-760 mm Hg) as illustrated in FIG. 1 and described above, for topical application to the cornea. In some embodiments, the oil comprises a perfluorocarbon or a silicon based oil approved for in vivo use in humans, or a mineral or vegetable oil of sufficient purity for human consumption. In some embodiments, the solution comprises an emulsion of oils approved for in vivo use in humans, with the aqueous portion of the emulsion containing catalase and/or iodide ion in sufficient quantity to decompose 20 micromolar of hydrogen peroxide within one minute. Examples of a suitable fluid include the synthetic oil perfluorodecalin, which in at least some cases carries 100 times more oxygen per volume than an aqueous saline solution. Perfluorodecalin is also UVA/blue light transparent, biologically inert and FDA approved for human use. One 5-micron tear film of perfluorodecalin pre-oxygenated at 300 mm Hg of oxygen could completely reoxygenate the entire stroma and still have a remaining partial pressure of oxygen equal to the partial pressure of oxygen in the atmosphere. The high partial pressure provides the driving force to transfer the oxygen into the stroma. In some embodiments, other oil based fluids which are used for this purpose include silicone oils that have 50-75 times the oxygen carrying capacity of saline, and olive oil or mineral oil with about 25 times the oxygen carrying capacity of saline. In some embodiments, these oils are used in pure form or as part of emulsions. In pure form, oils such as mineral oil float on top of the epithelial mucin layer if the epithelium is intact, or on top of Bowmans' membrane if the epithelium has been removed. Advantageously, these hydrophobic oil solutions do not migrate into the aqueous stroma. An additional advantage of the oxygenated oil fluids is that they all allow passage of CO₂ from the corneal stroma and do not absorb ions from the cornea that could upset the osmotic balance or thickness of the stroma. A known effect of applying oils onto the cornea is that oils can disrupt the barrier function of the epithelium. However, this barrier disruption is desirable in photochemical cross-linking, since it facilitates the infusion of riboflavin into the stroma.

In the above embodiments, the riboflavin and tear solutions are in gas permeable plastic bottles and must be used shortly after removal from the oxygen chamber to avoid or reduce oxygen loss. In alternative embodiments where storage and transport is required, the solutions are first oxygenated in any suitable manner, for example by bubbling pressurized oxygen through the solution. The oxygenated solutions are then stored in actinic or brown glass containers with suitable aluminum foil seals or gas-sealed caps to prevent equilibration with air.

### Ophthalmic Buffing

The time period for sufficient riboflavin to penetrate into the cornea without removal or treatment of the epithelium layer can be up to three hours. In some embodiments, eye surface buffing reduces the initial time needed for an ophthalmic solution (e.g., a riboflavin solution) to penetrate sufficiently into the cornea to as little as seven to eleven minutes without requiring removal of the epithelium, significantly reducing patient discomfort and the time needed to complete the treatment.

"Buffing" or preparation of the epithelium prepares it for improved penetration of the epithelium by the ophthalmic solution (e.g., a riboflavin solution), without having to remove the epithelium altogether. The cornea absorbs the riboflavin well until the corneal stroma is sufficiently loaded.

In the above embodiments, "buffing" or preparation of the epithelium along with use of the highly oxygenated tear and riboflavin solutions substantially increases delivery of both oxygen and riboflavin to the eye.

FIGS. 2 to 10 illustrate an exemplary device and method for improving the penetration of riboflavin or other topical fluids through the epithelial barrier, while avoiding the surgical complications that arise from deepithelialization, which in some embodiments results in more disruption of the tissue than is necessary for the procedure to be effective. The reduction in patient discomfort, the more rapid restoration of visual acuity, and the reduced risk of infection or stromal haze make a transepithelial procedure better for the patient.

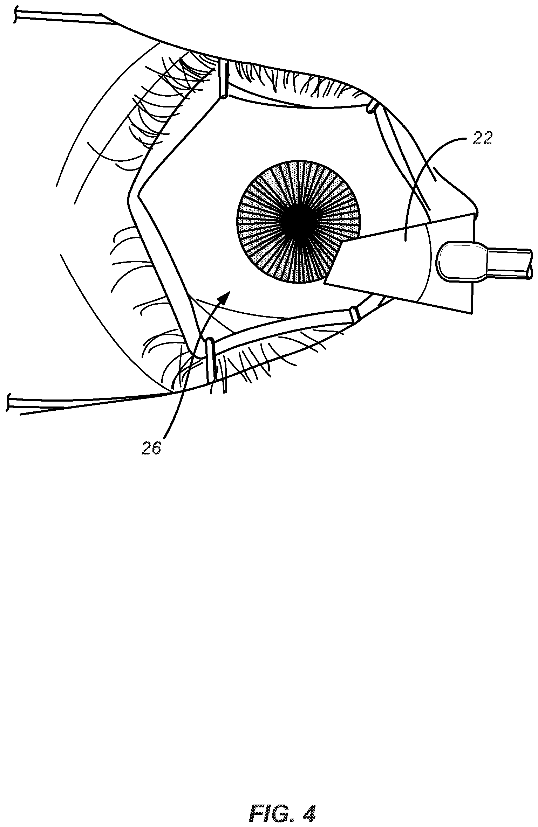

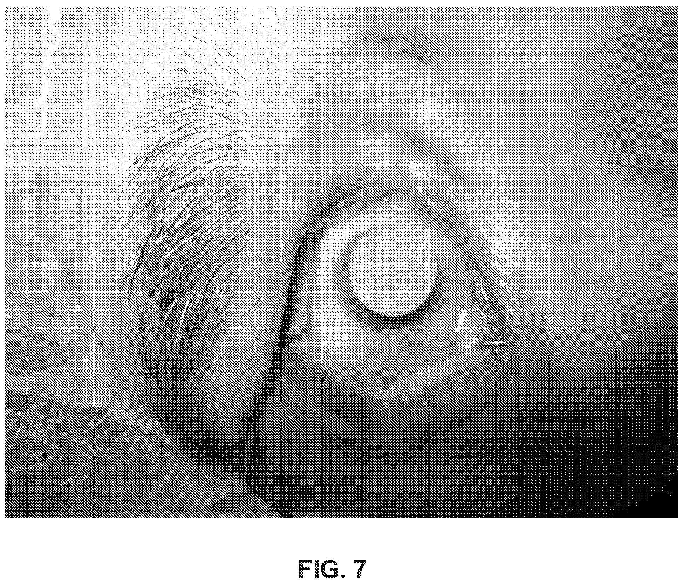

In some embodiments, a "sponge" device 20 as illustrated in FIG. 2 is rubbed gently over the surface of the eye 26, for example in a circular pattern, after application of a topical anesthetic, in order to "buff" the eye to a dull sheen, as illustrated in FIG. 4. In some embodiments, the sponge device 20 comprises a dry, relatively rigid sponge 22. In some embodiments, the sponge is secured to handle 25. In some embodiments, the sponge device is not secured to a handle. In some embodiments, the sponge 22 has an angled edge 24. In some embodiments, the sponge is round. In some embodiments, where the sponge is round, the sponge does not comprise a handle, for example as illustrated in Fig. 7. In some embodiments, the sponge edge induces microabrasion of the epithelium, with the abrasion process forming small microscratches on the epithelium to allow the riboflavin solution to penetrate through the epithelium into the cornea more easily. In some embodiments, buffing the eye removes lipids, mucus and microvilli as well as dead epithelial cells which would otherwise resist penetration of fluid through the epithelium.

In some embodiments, after buffing, an ophthalmic solution is applied to the eye in any suitable manner. In some embodiments, the ophthalmic solution is applied through a second, softer sponge placed over the eye. In some embodiments, the ophthalmic solution is dripped onto the sponge, or the sponge is pre-soaked with the ophthalmic solution. The second sponge acts as a reservoir to apply additional solution. In some embodiments, the ophthalmic solution is a riboflavin solution or artificial tear or a combination thereof. If the sponge is pre-loaded with riboflavin, it is stored in a dark packaging material prior to use to avoid or reduce light degradation. In some embodiments, the sponge is round. In some embodiments, the sponge is made from cellulose or any other suitable material. In some embodiments, the sponge is made of any fast wicking, lint-free material such as polyvinyl acetate, for example a round Merocel^{®} sponge which is also manufactured by Beaver-Visitec International, Inc.

In some embodiments, the sponge device comprises a wet sponge comprising an ophthalmic solution, rather than the dry sponge described above. In some embodiments, the wet sponge is rubbed gently over the surface of the eye, for example in a circular pattern. In some embodiments, a topical anesthetic is applied to the eye before the application and use of the wet sponge. In some embodiments, the wet sponge dvice induces microabrasion of the epithelium, with the abrasion process forming small microscratches on the epithelium to allow the riboflavin solution to penetrate through the epithelium into the cornea more easily. In some embodiments, buffing the eye removes lipids, mucus and microvilli as well as dead epithelial cells which would otherwise resist penetration of fluid through the epithelium.

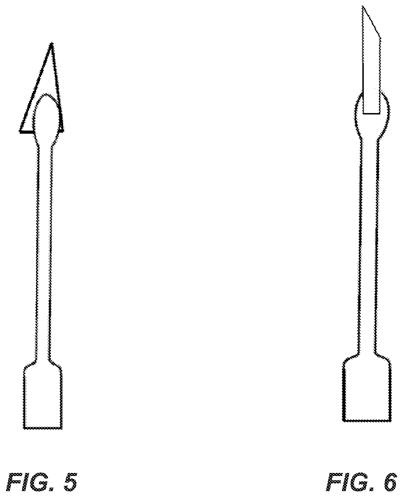

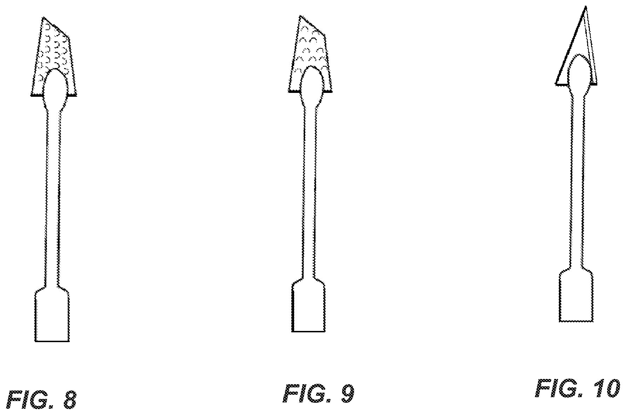

Any suitable sponge shape or material is contemplated for use with the methods disclosed herein, see e.g., FIGs. 2, 3A, 3B, 4, 5, 6, 7, 8, 9, 10. In some embodiments, the sponge is round, see e.g., Figure 7. In some embodiments, the sponge does not have a pointed tip. In some embodiments, the sponge does not have sharp edges. In some embodiments, the sponge has a pointed tip, for example the sponge is spear-tipped or arrow shaped. In some embodiments, the sponge has a feathered tip. In some embodiments, the sponge dvice has a pointed tip, for example the sponge is trapezoidal-shaped. In some embodiments, the sponge has a beveled edge, see e.g., FIGs 6 and 10. In some embodiments, the sponge has a 10 to 80 degree beveled edge, see e.g., FIG. 7 and FIG. 10. In some embodiments, the sponge has flat edges.

Where the sponge is a round sponge, the sponge operatively covers all or a portion of the eye or the cornea. In some embodiments, the size of the sponge does not exceed the size of the eye or the cornea. In some embodiments, the diameter of the sponge is about 3mm- about 12mm. In some embodiments, the sponge is about 1 mm - about 5 mm in thickness.

In some embodiments, the sponge has a pointed tip. In some embodiments, the pointed tip is used for wicking purposes. In FIG. 2, the sponge is specifically modified to have a flat angled edge 24 suitable for the circular pattern, buffing procedure described above.

In some embodiments, the sponge has a non-pointed end edges 28 and 29. Such sponges are illustrated in FIG. 3A and 3B.

In some embodiments, a suitable sponge which is used for the buffing procedure is made from a relatively rigid, highly absorbent cellulose or similar material, for example a Weck-Cel^{®} sponge as manufactured by Beaver-Visitec International, Inc. of Waltham, MA.

In some embodiments, the sponge further comprises microfilaments, for example the microfilaments are embedded within the sponge. In some embodiments, the sponge does not comprise microfilaments. In some embodiments, the microfilaments are made of any suitable strong, flexible polymer. In some embodiments, the microfilaments are made of polyester, or polyamide. In some embodiments, the microfilaments are arranged in an ordered pattern. In some embodiments, the microfilaments are vertically oriented, see e.g., FIG. 8. In some embodiments, the microfilaments are horizontally oriented, see e.g., FIG. 9. In some embodiments, the microfilaments are not arranged in an ordered pattern. In some embodiments, the microfilaments are arranged in a disordered pattern.

Specific embodiments include:
1. A composition for promoting photochemical cross-linking in eye tissue, the composition comprising: 0.2% to 10.0% by weight riboflavin in an aqueous carrier.
2. The composition of embodiment 1, wherein the composition comprises 0.2% to 5.0% by weight riboflavin.
3. The composition of embodiment 1, wherein the composition comprises 1.0% to 2.0% by weight riboflavin.
4. The composition of embodiment 1, further comprising 0.001% to 6.0% sodium iodide or catalase by weight.
5. The composition of embodiment 4, wherein the iodide is in its ionized (I⁻) form.
6. The composition of embodiment 1, having a pH from about 7.0 to about 8.4.
7. The composition of embodiment 1, wherein the composition is a dry powder.
8. The composition of embodiment 1, stored in an oxygen permeable plastic container.
9. The composition of embodiment 8, wherein the oxygen permeable plastic container comprises polyurethane or low density polyethylene.
10. The composition of embodiment 1, further comprising pressurized oxygen.
11. The composition of embodiment 10, comprising 350 to 500 Torr oxygen.
12. The composition of embodiment 1, further comprising a reducing agent.
13. The composition of embodiment 12, wherein the reducing agent comprises one or more of sodium thiosulfate, vitamin C, and sodium bisulfate.
14. A method for enhancing photochemical cross-linking in eye tissue, comprising: introducing to the eye tissue a composition comprising 0.2% to 10.0% by weight riboflavin in an aqueous carrier.
15. The method of embodiment 14, wherein the composition comprises 0.2% to 5.0% by weight riboflavin.
16. The method of embodiment 14, wherein the composition comprises 1.0% to 2.0% by weight riboflavin.
17. The method of embodiment 14, further comprising administering 0.001% to 6.0% by weight sodium iodide or catalase, artificial tears, or any combinations thereof.
18. The method of embodiment 14, wherein the composition has a basic pH.
19. The method of embodiment 18, wherein the basic pH is from about 7.0 to about 8.4.
20. The method of embodiment 14, wherein the composition is a dry powder.
21. The method of embodiment 14, further comprising keeping the composition in an oxygen permeable plastic container.
22. The method of embodiment 21, wherein the oxygen permeable plastic container comprises polyurethane or low density polyethylene.
23. The method of embodiment 21, further comprising placing the oxygen permeable plastic container in an oxygen chamber to be supplied and saturated with pressurized oxygen.
24. The method of embodiment 14, further comprising saturating the composition with oxygen.
25. The method of embodiment 24, wherein the composition is saturated with oxygen to have an oxygen content of
26. The method of embodiment 14, wherein the composition further comprises a reducing agent.
27. The method of embodiment 26, wherein the reducing agent comprises one or more of sodium thiosulfate, vitamin C, and sodium bisulfate.
28. A method of applying an ophthalmic composition to an eye, comprising applying the ophthalmic composition after buffing the epithelium of an eye.
29. The method of embodiment 28, comprising buffing the epithelium of the eye by contacting the eye with a sponge.
30. The method of embodiment 28, comprising buffing the epithelium of the eye by rubbing the eye with a sponge.
31. The method of embodiment 30, comprising buffing the epithelium of the eye by rubbing the eye with a sponge in a circular pattern.
32. The method of embodiment 28, comprising removing lipids, mucus and microvilli.
33. The method of embodiment 28, wherein the sponge is dry or comprises an ophthalmic composition.
34. The method of embodiment 33, wherein the sponge comprises a riboflavin composition, artificial tears, or a combination thereof.
35. The method of embodiment 28, wherein the sponge is round.
36. The method of embodiment 28, further comprising applying the ophthalmic composition with a second sponge.
37. The method of embodiment 36, comprising placing the second sponge over the eye to act as a depot or reservoir for the ophthalmic composition.
38. A round sponge device, comprising a sponge which operatively covers all or a portion of the eye.
39. The sponge device of embodiment 38, wherein the size of the sponge does not exceed the size of the eye.
40. The sponge device of embodiment 38, wherein the diameter of the sponge is about 3mm- about 12mm.
41. The sponge device of embodiment 38, further comprising 0.2% to 10.0% by weight riboflavin in an aqueous carrier, and optionally, sodium iodide, catalase, artificial tears, or any combinations thereof.
42. The sponge device of embodiment 38, further comprising microfilaments.
43. The sponge device of embodiment 38, made of cellulose or polyvinyl acetate.
44. The sponge device of embodiment 38, further comprising a handle operatively connected to the sponge.
45. The sponge device of embodiment 38, wherein the sponge does not comprise a handle.
46. A sponge device for use in introducing microabrasions into an eye, comprising a sponge having a surface shaped for rubbing across the surface of an eye.
47. The sponge device of embodiment 46, wherein the sponge has little or no risk of disrupting or perforating the epithelium.
48. The sponge device of embodiment 46, wherein the sponge is round.
49. The sponge device of embodiment 46, wherein the sponge does not have a pointed tip.
50. The sponge device of embodiment 46, wherein the sponge does not have sharp edges.
51. The sponge device of embodiment 46, wherein the sponge has a pointed tip.
52. The sponge device of embodiment 46, wherein the sponge is spear-tipped or arrow shaped.
53. The sponge device of embodiment 46, wherein the sponge has a feathered tip.
54. The sponge device of embodiment 46, wherein the sponge has a beveled edge.
55. The sponge device of embodiment 54, wherein the sponge has a 10 to 80 degree beveled edge.
56. The sponge device of embodiment 46, wherein the sponge has flat edges.
57. The sponge device of embodiment 46, wherein the sponge is made of a cellulose or polyvinyl acetate.
58. The sponge device of embodiment 46, wherein the sponge further comprises microfilaments.
59. The sponge device of embodiment 58, wherein the microfilaments are vertically oriented.
60. The sponge device of embodiment 58, wherein the microfilaments are horizontally oriented.
61. The sponge device of embodiment 58, wherein the microfilaments are not arranged in an ordered pattern.
62. The sponge device of embodiment 58, wherein the microfilaments are arranged in a disordered pattern.
63. The sponge device of embodiment 46, wherein the sponge does not comprise microfilaments.
64. The sponge device of embodiment 46, further comprising a handle operatively connected to the sponge.
65. The sponge device of embodiment 46, wherein the sponge device does not comprise a handle.
66. The sponge device of embodiment 46, further comprising 0.2% to 10.0% by weight riboflavin in an aqueous carrier, and optionally, sodium iodide, catalase, artificial tears, or any combinations thereof.

### Example

Two formulations of riboflavin solution were tested to determine saturation time. Formulation 1 had a riboflavin concentration of 0.1%. Formulation 2 had a riboflavin concentration of 0.5%.

Saturation was determined using "serial slit-lamp assessments" of the cornea at approximately 5 minute intervals. Riboflavin has a characteristic green color when illuminated with visible light. Slit-lamp assessments using visible light reveal the depth and uniformity of riboflavin throughout the corneal thickness.

### Outcome measures:

UCVA, BSCVA, and K Max at 6 months and 1 year follow-up visits.

### Inclusion Criteria:

Patients who had undergone trans-epithelial cross-linking in one or both eyes were included in the analysis. Patients with a diagnosis of keratoconus or post-LASIK ectasia were included in this analysis.

### Exclusion Criteria:

Patients with previous RK, INTACS, more than one cross-linking procedure per eye, and/or patients who were pseudo-phakic or had a diagnosis of nuclear sclerotic cataract were excluded from this analysis

### Results - Formulation 1, 0.1 % Riboflavin

Loading Time with formulation 1 ranged from 30 minutes minimum to 100 minutes maximum, with an average loading time of 56.33 minutes.

Results are presented in Table 1.

**Table 1**

| | | **6 month** | **1 year** |
|---|---|---|---|
| Average Follow Up Time | | 6.67 months | 11.84 months |
| Minimum and Maximum Follow up Time | | 4 months, 9 months | 9 months, 15 months |
| UCVA | Improved 1 or more lines | 54% | 61% |
| | Worsened | 10% | 12% |
| | No Change | 36% | 27% |
| BSCVA | Improved 1 or more lines | 48% | 54% |
| | Worsened | 13% | 15% |
| | No Change | 39% | 31% |
| K Max (Avg. change from Pre-op) | | 0.44D flattening | 0.65D flattening |

### Results - Formulation 2, 0.5% Riboflavin

Loading Time with formulation 2 ranged from 8 minutes minimum to 60 minutes maximum, with an average loading time of 22.17 minutes

Results are presented in Table 2.

**Table 2**

| | | **6 month** | **1 year** |
|---|---|---|---|
| Average Follow Up Time | | 5.89 months | 11.33 months |
| Minimum and Maximum Follow up Time | | 4 months, 9 months | 9 months, 13 months |
| UCVA | Improved 1 or more lines | 49% | 67% |
| | Worsened | 11% | 13% |
| | No Change | 41% | 20% |
| BSCVA | Improved 1 or more lines | 48% | 47% |
| | Worsened | 14% | 7% |
| | No Change | 38% | 40% |
| K Max (Avg. change from Pre-op) | | 1.36D flattening | 2.29D flattening |

The above description of the disclosed embodiments to enable any person skilled in the art to make or use the invention. Various modifications to these embodiments will be readily apparent to those skilled in the art, and the generic principles described herein can be applied to other embodiments without departing from the spirit or scope of the disclosure. Thus, it is to be understood that the description and drawings presented herein represent an exemplary embodiment of the disclosure and are therefore representative of the subject matter which is broadly contemplated by the present invention. It is further understood that the scope of the present disclosure fully encompasses other embodiments that may become obvious to those skilled in the art and that the scope of the present disclosure is accordingly not limited.

## Claims

1. A composition for use in promoting photochemical cross-linking in eye tissue, the composition comprising 0.2% to 10% by weight riboflavin and 0.001% to 6.0% by weight sodium iodide in an aqueous carrier.

2. The composition for use of claim 1, wherein the composition comprises 0.2% to 5% by weight riboflavin.

3. The composition for use of claim 1, wherein the composition comprises 1.0% to 2.0% by weight riboflavin.

4. The composition for use of claim 1, having a pH from about 7.0 to about 8.4.

5. The composition for use of claim 1, further comprising a reducing agent, optionally wherein the reducing agent comprises one or more of sodium thiosulfate, vitamin C, and sodium bisulfate.

6. The composition for use of claim 1, wherein the riboflavin is riboflavin-5'-phosphate.

7. The composition for use of any one of claims 1-6, wherein the composition is for application to the eye with a sponge pre-soaked with the composition, wherein the sponge acts as a reservoir for the composition, optionally wherein the sponge is made of any fast wicking, lint-free material.

8. A sponge for applying the composition of any one of claims 1-6 to an eye, wherein the sponge is pre-soaked with the composition and acts as a reservoir for the composition.

9. A round sponge device, comprising a sponge which operatively covers all or a portion of the eye.

10. The sponge device of claim 9, wherein:
(a) the size of the sponge does not exceed the size of the eye;
(b) diameter of the sponge is about 3mm- about 12mm;
(c) the sponge device comprises 0.2% to 10.0% by weight riboflavin in an aqueous carrier, and optionally, sodium iodide, catalase, artificial tears, or any combinations thereof; or
(d) the sponge is made of a fast wicking, lint-free material.

11. A sponge device for use in introducing microabrasions into an eye, comprising a sponge having a surface shaped for rubbing across the surface of an eye.

12. The sponge device of claim 11, wherein:
(a) the sponge has little or no risk of disrupting or perforating the epithelium;
(b) the sponge is round;
(c) the sponge does not have sharp edges; or
(d) the sponge has flat edges.

13. The sponge device of any one of claims 9-12, wherein:
(a) the sponge is made of a cellulose or polyvinyl acetate; or
(b) the sponge further comprises microfilaments.

14. The sponge device of claim 11, further comprising a handle operatively connected to the sponge.

15. A system comprising:
(a) the sponge of claim 8 or the round sponge device of any one of claims 9, 10 or 13; and
(b) the sponge device of any one of claims 11-14.
